# EUROPÄISCHE PATENTSCHRIFT

(11) **EP 2 090 332 B1**
(45) Veröffentlichungstag und Bekanntmachung des Hinweises auf die Patenterteilung: **26.02.2014**
(21) Anmeldenummer: 09150823.4
(22) Anmeldetag: 19.01.2009
(51) Int. Cl.: A61N 1/05, H01R 13/52

(54) **Elektrodenleitung und Anschlussstück für elektromedizinische Implantate**
Electrode line and connecting piece for electromedical Implants
Combinaison synergétique d'un ou plusieurs fongicides contenant des éthers glycoliques

(30) Priorität: 13.02.2008 DE 102008008927
(43) Veröffentlichungstag der Anmeldung: 19.08.2009
(60) Teilanmeldung: 13196928.9
(73) Patentinhaber: Biotronik CRM Patent AG, 6341 Baar (CH)
(72) Erfinder: Günther, Thomas, 14552, Michendorf OT Wilhelmshorst (DE); Bartels, Klaus, 10115, Berlin (DE); Steglich, Carsten, 12587, Berlin (DE)
(74) Vertreter: Lindner-Vogt, Karin L.

(56) Entgegenhaltungen:
- US-A1- 2005 182 470
- US-A1- 2005 221 671
- US-A1- 2006 259 106

## Beschreibung

Die Erfindung betrifft eine Elektrodenleitung zum Anschluss an elektromedizinische Implantate wie beispielsweise einen Herzschrittmacher, einen Cardioverter/Defibrillator oder einen Neurostimulator oder geeignete Kombinationen daraus. Insbesondere betrifft die Erfindung ein Anschlussstück für eine solche Elektrodenleitung.

Elektromedizinische Implantate der genannten Art sind in verschiedenen Ausführungen grundsätzlich bekannt. Derartige Implantate weisen üblicherweise ein dichtes Gehäuse auf, welches eine Stromversorgung und weitere elektronische und elektrische Bauteile umfasst, um Stimulationsimpulse und/oder Defibrillationsschocks zu erzeugen und über eine lang gestreckte Elektrodenleitung mit einer Längsachse an bestimmte Körperstellen wie eine oder mehrere Kammern eines Herzens, einer Nervenbahn, im Rückenmark oder im Gehirn abzugeben oder um elektronische Potentiale dieser Körperstellen zu erfassen und zu verarbeiten. Zu diesem Zweck sind solche Implantate üblicherweise mit Elektrodenleitungen verbunden, die mit einem Anschlussstück am proximalen Ende der Elektrodenleitung in eine Buchse in einem sogenannten Header des jeweiligen Implantats eingesteckt sind. Sowohl das Anschlussstück als auch die Buchse im Header des Implantats weisen miteinander korrespondierende elektrisch leitende Kontakte auf, um eine elektrisch leitende Verbindung zwischen dem Herzstimulator und der Elektrodenleitung herzustellen.

Neben einem Anschlussstück am proximalen Ende umfassen derartige Elektrodenleitungen weiter einen tubularen, flexiblen und biegbaren Elektrodenkörper mit einer Längsachse und einem distalen Ende, welches der Therapiestelle zugewandt ist und an dem sich Elektrodenpole befinden, über die therapeutische und/oder diagnostische Signale empfangen oder abgegeben werden. Die Elektrodenpole können dabei die Form von Spitzenelektroden am distalen Ende oder Ringelektroden in der Nähe des distalen Endes oder aber auch großflächige Wendelabschnitte, die zur Abgabe von Schocks dienen, annehmen.

Der Elektrodenkörper weist eine elektrisch isolierende, vorzugsweise biokompatible, Außenhülle auf, in welcher die Zuleitungen eingebettet sind, um die Zuleitungen zu schützen und gegenüber dem Körpergewebe zu isolieren. Das Material einer solchen Hülle kann beispielsweise Silikon sein. Zusätzlich kann eine weitere äußere Beschichtung auf der isolierenden Außenhülle aufgebracht sein, welche ebenfalls biokompatibel sein kann, einen geringen Reibungskoeffizienten aufweist und das Platzieren der Elektrodenleitung erleichtert. Diese Anforderungen für die äußere Beschichtung erfüllt beispielsweise Polyrethan.

Die beschriebenen Zuleitungen dienen zur Übertragung von Messsignalen von den Elektrodenpolen an das Anschlussstück oder von Stimulationsimpulsen oder Defibrillationsschocks vom Anschlussstück zu den jeweiligen Elektrodenpolen. Eine oder mehrere der beschriebenen Zuleitungen können eine gewendelte Form aufweisen, welche innerhalb der Außenhülle verläuft. Durch diese gewendelte Zuleitung, deren Längsachse auf der Längsachse der Elektrodenleitung liegt, gewinnt die Elektrode elastische Eigenschaften, was bei einer eventuellen mechanischen Zugbelastung verhindert, dass die Elektrode reißt oder die elektrischen Zuleitungen beschädigt werden. Weiterhin bildet diese gewendelte Zuleitung ein Lumen vom proximalen zum distalen Ende der Elektrodenleitung aus, entlang welchem die Elektrode an einem Führungsdraht oder Mandrin geführt werden kann, durch das aber auch therapeutische Stoffe wie beispielsweise Medikamente geleitet werden können.

Weiterhin weist die Elektrodenleitung am Übergang zum Anschlussstück einen Knickschutz auf, der einerseits das Abknicken des Anschlussstückes verhindert und andererseits als Zugentlastung für gefügte Kontakte zwischen Elektrodenzuleitung und Anschlussstück dient.

Die vorliegende Erfindung gilt dem Anschlussstück am proximalen Ende der Elektrodenleitung und dessen Verbindung zu übrigen Elektrodenleitungen.

Das hier betroffene Anschlussstück ist dabei von der Art, die mehrere, ringförmige Kontakte gleichen Außendurchmessers aufweist und der zukünftigen Norm IS-4 entspricht. Eine Variante eines derartigen Anschlussstücks ist beispielsweise in US 2005/0221671 beschrieben. Dort ist ein Anschlussstück für eine Elektrodenleitung zum Anschluss an ein elektrisches Implantat offenbart, das eine lang gestreckte Form in einer Längsrichtung und ein proximales und ein distales Ende aufweist. Dieses Anschlussstück umfasst eine elektrisch leitende Steckerbaugruppe, die in Längsrichtung proximal aus dem proximalen Ende des Anschlussstückes ragt.
Weiterhin umfasst das Anschlussstück mehrere ringförmige, elektrisch leitende Kontakte gleichen Außendurchmessers mit in Längsrichtung dazwischen angeordneten Isolationsabschnitten desselben Außendurchmessers, wobei die Isolationsabschnitte die elektrischen Kontakte derart fixieren, dass ihre Lage in Längsrichtung unveränderlich sind, wobei die elektrischen Kontakte mit jeweils einer korrespondierenden elektrischen Zuleitung des Elektrodenkörpers verbunden ist.
Ebenso umfasst das Anschlussstück aus dem Stand der Technik ein Stützelement mit einem im Wesentlichen kreisrunden Außenquerschnitt und mit einer Längsachse entlang der Längsrichtung, welches ausgebildet ist, die Isolationsabschnitte durch Vorsprünge zu tragen und in Radialrichtung zu fixieren, und welches eine auf seiner Längsachse befindlichen durchgehenden Bohrung aufweist, welche die Steckerbaugruppe und/oder eine Zuleitung des Elektrodenkörpers aufnimmt.

Als "Außenquerschnitt" im Sinne der Erfindung wird der Grundkreis eines Hüllzylinders verstanden, auf dem die äußeren Kanten oder Flächen des Stützelementes liegen. Der Außenquerschnitt wird dabei aber nicht vom Anschlag gebildet, der über den Außenquerschnitt hinausragt.

Durch die Vorsprünge im Stützelement des Standes der Technik, die sich nur in einer Ebene in Radialrichtung befinden, ist es jedoch nicht möglich, die Isolationsabschnitte und die elektrisch leitenden Kontakte zuverlässig so zu fixieren, dass eine Verschiebung in Radialrichtung nicht möglich ist. Ein Verkippen der Isolationsabschnitte von der Längsrichtung/Längsachse weg ist also möglich. Zudem ist eine ökonomische Vorfertigung des Anschlussstückes nicht möglich, da sich Elemente des Elektrodenkörpers, an dessen proximalen Ende das Anschlussstück angebracht wird, in das Anschlussstück erstrecken, um beispielsweise Steckerbaugruppe mit einer elektrisch leitenden Wendel zu verbinden.

Die europäische Patentanmeldung 07023315.0 der Anmelderin behebt die genannten Nachteile und beschreibt eine Elektrodenleitung mit einem solchen Anschlussstück, das mehrere ringförmige, elektrisch leitende Kontakte gleichen Außendurchmesser mit in Längsrichtung dazwischen angeordneten Isolationsabschnitten desselben Außendurchmesser aufweist, von denen die elektrisch leitenden Kontakte mit jeweils einer elektrisch leitenden Zuleitung elektrisch leitend verbunden sind. Das gezeigte Anschlussstück ist von mehreren isolierenden Zwischenstücken und von, von diesen gehaltenen, elektrischen Kontakten mit daran befestigten Anschlussleitungen gebildet, in Längsrichtung des Anschlussstücks zusammengesteckt und nach dem Zusammenstecken mit isolierendem Kunststoff umspritzt.

Dieses Anschlussstück hat jedoch den Nachteil, dass die isolierenden Zwischenstücke innen liegen. Die Spritzmasse, die durch äußeres Umspritzen zwischen den elektrisch leitenden Kontakten angeordnet ist, kann nicht die geforderte Hochspannungs- Überschlagfestigkeit bereitstellen, weshalb es immer wieder zu fehlerhaften Übertragungen von Signalen, Impulsen oder Defibrillationsschocks kommt.

Daher ist es Aufgabe der Erfindung, ein Anschlussstück, eine Elektrodenleitung mit einem solchen Anschlussstück und ein Verfahren zur Herstellung eines solchen Anschlussstückes und einer Elektrodenleitung mit einem solchen Anschlussstück zu schaffen, das die Nachteile des Standes der Technik behebt, eine Fertigung mit vertretbaren Aufwand (beispielsweise durch eine Erhöhung des Vormontagegrades) unabhängig von der Art und Zahl der Kontakte der Elektrodenleitung ermöglicht und die geforderte Hochspannungs- Überschlagfestigkeit gewährleistet.

Die Aufgabe wird durch die Vorrichtungen aus Anspruch 1 und 12 und die Fertigungsverfahren aus Anspruch 17 und 18 gelöst.

Das erfindungsgemäße Anschlussstück weist ein Stützelement mit n in Längsrichtung beabstandete umlaufende Nuten, welche ausgebildet sind, eine Spritzmasse aufzunehmen, und n sich vom proximalen bis zum distalen Ende parallel der Längsachse erstreckende Aussparungen, um Anschlussleitungen von den elektrisch leitenden Kontakten zum distalen Ende zu führen, auf, wobei n der Anzahl der elektrisch leitenden Kontakte entspricht.

Die Anzahl "n" bezeichnet dabei eine natürliche ganze Zahl.

Vorzugsweise ragen die elektrischen Anschlussleitungen distal aus dem distalen Ende des Anschlussstücks heraus, um eine leichte Zugänglichkeit und ein sicheres elektrisches und mechanisches Verbinden mit einem Elektrodenkörper zu ermöglichen.

Dies ermöglicht eine einfache Vormontage des Anschlussstückes, da alle wesentlichen Elemente wie die elektrisch leitenden Kontaktflächen und die Isolationsabschnitte durch die Spitzmasse zusammengefügt werden können. Die notwendige elektrische Verbindung mit dem Elektrodenkörper kann einerseits durch die Anschlussleitungen, die am distalen Ende des Anschlussstückes, an dem der Elektrodenkörper angebracht werden kann, in distaler Richtung herausragen und die mit den elektrischen Zuleitungen des Elektrodenkörpers elektrisch verbunden werden. Die sichere mechanische Befestigung erfolgt durch die direkt am Stützelement angebrachte und befestigte isolierende Außenhülle des Elektrodenkörpers, die dadurch gleichzeitig als Zugentlastung der Verbindungsstelle zwischen den elektrischen Anschlussleitungen des Anschlussstückes und den elektrischen Zuleitungen des Elektrodenkörpers dient.

Bevorzugt schließen die elektrisch leitenden Kontakte und die Isolationsabschnitte einerseits und das Stützelement andererseits einen Innenraum ein, in den die Spritzmasse nach dem Zusammenfügen der elektrisch leitenden Kontakte, der Isolationsabschnitte und des Stützelements durch Spritzgießen eingebracht ist und so die Kontakte, Isolationsabschnitte und das Stützelement fest miteinander verbindet.

Die entlang der Längsachse verlaufenden Aussparungen sind dabei so angelegt, dass sie entlang des gesamten Stützelementes radial nach außen offen sind. Das heißt, sie sind vor der Montage freiliegend, so dass leicht die elektrischen Anschlussleitungen eingebracht werden können. Nach der Montage werden die Aussparungen von den ringförmigen elektrisch leitenden Kontakten und den Isolationsabschnitten bedeckt, so dass sich ein geschlossener Kanal mindestens vom jeweiligen elektrisch leitenden Kontakt zum distalen Ende bildet, in dem die Anschlussleitungen geschützt von den jeweiligen Kontakten zum distalen Ende des Anschlussstückes verlaufen. Weiterhin verlaufen die Anschlussleitungen dann isoliert in diesen Aussparungen. Da das Stützelement bevorzugt aus einem Polymer, besonders bevorzugt aus PEEK (Poly Ether Ether Keton), welches unter dem Handelsnamen "Victrex" von der Firma Optima vertrieben wird, aus PEKK (Poly Ether Keton Keton), welches unter dem Handelsnamen "OXPEKK" von der Firma Oxford Performance Materials Inc. Vertrieben wird, oder aus POM (Polyoxymethylen), welches unter dem Handelsnamen "Hostaform" von der Firma Ticona vertrieben wird, oder weiter bevorzugt aus Keramiken hergestellt ist, liegt somit eine komplette isolierende Umgebung um die Anschlussleitung vor.

Die Begrenzungen der Aussparungen in Richtung Stützelement - also Richtung Längsachse des Anschlussstückes - sind so gestaltet, dass einerseits die Montage von außen leicht möglich ist und andererseits die Anschlussleitungen so sicher zum Liegen kommen, dass sie während der Montage nicht verrutschen. Weiterhin richtet sich die Form an der Anzahl n der elektrisch leitenden Kontakte und derselben Anzahl an Anschlussleitungen. So sind die Begrenzungen der Aussparungen in Richtung Längsachse bevorzugt durch Sekantenfläche begrenzt, so dass das Stützelement einen Körper mit einem polyedrischen Querschnitt, vorzugsweise mit einem gleichseitigen N-Eck mit einem Hüllkreis als Außenquerschnitt, aufweist. Besonders bevorzugt sind drei Aussparungen mit drei Sekantenflächen. Die Begrenzungen der Aussparungen in Richtung Stützelement können aber auch andere Formen wie u- oder v-förmige Begrenzungen annehmen, damit mehr als drei Anschlussleitungen zum distalen Ende geführt werden können.

Ein Polyeder (auch Vielflach, Vielflächner oder Ebenflächner) im Sinne der Erfindung ist ein lang gestreckter Körper mit einer Querschnittsfläche mit mehreren gewinkelt angeordnete Strecken, wobei die sich bildenden Kanten und Flächen von der Längsachse maximal von einem Hüllkreis als Außenquerschnitt begrenzt wird. Erfindungsgemäß können auch Abschnitte des Polyeders durch nicht geradlinige Strecken, sondern durch Hüllkreisabschnitte gebildet werden, die dann eine Passung für die elektrischen Isolierungen und/oder elektrisch leitenden Kontakte des Anschlussstückes bilden.

Das Anschlussstück kann bevorzugt ein Stützelement mit einem umlaufenden Anschlag am distalen Ende oder in dessen Nähe aufweisen, um die exakte Montage der elektrisch leitenden Kontakte und Isolationsabschnitte zu ermöglichen. Durch den Anschlag können die Kontakte und Isolationsabschnitte abwechselnd so aufgeschoben und positioniert werden, dass sie normgerecht vormontiert werden können. Der Anschlag weist dazu eine Aussparung auf, die entlang der Längsachse durch den Anschlag führt. Diese Aussparung kann dabei entweder weiter nach außen offen sein oder aber durch eine Bohrung gebildet sein, durch die die Anschlussleitungen geführt sind.

Um das Einspritzen der Spritzmasse zu vereinfachen und eine homogene Verteilung der Spritzmasse zu ermöglichen, ist der von den elektrisch leitenden Kontakten und den Isolationsabschnitten einerseits und dem Stützelement andererseits eingeschlossene Innenraum zusätzlich durch umlaufende Nuten und die sich vom proximalen bis zum distalen Ende parallel der Längsachse erstreckende Aussparungen des Stützelements gebildet. Dadurch werden die Aussparungen miteinander verbunden und die Spritzmasse kann mittels eines Spritzvorgangs eingebracht werden. Dies führt zu einer ökonomischeren Herstellung, da der Innenraum mit nur einem Spritzvorgang komplett ausgegossen werden kann. Die Spritzmasse kann bevorzugt aus einem einzigen Material, beispielsweise Polyurethan, PEEK (Poly Ether Ether Keton), welches unter dem Handelsnamen "Victrex" von der Firma Optima vertrieben wird, PEKK (Poly Ether Keton Keton), welches unter dem Handelsnamen "OXPEKK" von der Firma Oxford Performance Materials Inc. vertrieben wird, oder aus POM (Polyoxymethylen), welches unter dem Handelsnamen "Hostaform" von der Firma Ticona vertrieben wird, oder weiter bevorzugt aus Keramiken oder besonders bevorzugt aus denselben Materialien wie das Stützelement bestehen. Alternativ kann die Spritzmasse wenigstens aus zwei der genannten Materialien bestehen.

Bevorzugt sind die umlaufenden Nuten so gegeneinander beabstandet, dass die in Längsrichtung vor und hinter den Nuten liegenden umlaufenden Rippen die Isolationsabschnitte tragen und einen Außendurchmesser aufweisen, der einen passgenauen Sitz für den jeweiligen Isolationsabschnitt ermöglicht. Die Rippen weisen bevorzugt eine Breite von 1 bis 6 mm, besonders bevorzugt zwischen 1,5 und 5,25 mm auf.

Die Steckerbaugruppe kann bevorzugt aus einem Steckerpin und einem Kontaktstift, der bevorzugt eine lang gestreckte rotationssymmetrische Form mit einer Längsachse aufweist, die auf der Längsachse des Anschlussstückes zum Liegen kommt, bestehen. Dabei dient der Steckerpin zum Kontaktieren mit dem elektrischen Implantat, und der Kontaktstift zum Herstellen einer elektrisch leitenden Verbindung mit einer Zuleitung des Elektrodenkörpers. Der Steckerpin ist bevorzugt über ein Schweißverfahren (beispielsweise durch Laserstrahl- oder Widerstandsschweißverfahren am Kontaktstift fest fixiert. Auf der distalen, dem Elektrodenkörper zugewandten Seite des Kontaktstiftes weist der Kontaktstift eine Vertiefung entlang der Längsachse des Kontaktstiftes auf, mittels der eine Zuleitung des Elektrodenkörpers - bevorzugt die Wendelförmige - fest angebracht werden kann. Die Befestigung ist bevorzugt durch einen Crimpvorgang oder einen Schweißvorgang (beispielsweise durch Laserstrahl- oder Widerstandsschweißen) erfolgt.

Vorzugsweise weist die Steckerbaugruppe ein Widerlager auf, welches am Kontaktstift beispielsweise fest mittels eines der soeben genannten Befestigungsverfahren verbunden ist. Weiterhin weist das Stützelement mindestens am proximalen Ende ein Innengewinde auf, in das eine Hohlschraube in distaler Richtung eingeschraubt werden kann. Diese Hohlschraube verhindert im eingeschraubten Zustand durch Zusammenwirken mit dem Widerlager, dass die Steckerbaugruppe nicht in proximaler Richtung aus dem Anschlussstück rutschen kann. Bevorzugt handelt es sich bei der Hohlschraube um eine Verschlussschraube, die sich näher am proximalen Ende des Anschlussstückes befindet als das Widerlager an der Steckerbaugruppe. Die Hohlschraube weist dabei eine Bohrung auf, deren Durchmesser an den Durchmesser des Kontaktstiftes angepasst ist, die einen geringeren Durchmesser als das Widerlager und der Steckerpin hat. Dadurch wirken die Schraube und das Widerlager derart zusammen, dass ein Herausrutschen der Steckerbaugruppe aus dem Anschlussstück in proximaler Richtung verhindert wird. Mit dem Steckerpin wirkt die Hohlschraube derart zusammen, dass die Steckerbaugruppe nicht in distaler oder proximaler Richtung rutschen kann.

Der Vorteil dieser Konstruktionsweise ist, dass die Steckerbaugruppe ohne Beschädigung mehrfach in die Steckerbuchse des Anschlussgehäuses eines elektrischen Implantats eingesteckt und wieder entfernt werden kann.
Weiterhin ist von Vorteil, dass die Steckerbaugruppe frei um die Längsachse rotierbar ist. Diese drehbare Steckerbaugruppe wird bei Elektrodenleitungen häufig benötigt, welche eine so genannte aktive Fixierung am distalen Ende aufweisen. Eine aktive Fixierung ist meist als eine Befestigungswendel ausgeführt - bevorzugt als Fixierungsschraube. Um diese Fixierungsschraube im Gewebe des Herzens zu befestigen, muss eine Rotationsbewegung ausgeführt werden können. Die Elektrodenleitung als Ganzes kann nicht gedreht werden, weshalb die Steckerbaugruppe und die daran befestigte gewendelte Zuleitung drehbar (rotierbar) um die Längsachse gelagert sind. Die gewendelte Zuleitung wiederum ist mit der aktiven Fixierung verbunden. Eine rotatorische Bewegung an der Steckerbaugruppe bewirkt also die Fixierung im Herzgewebe, indem die Befestigungswendel aus der Elektrode heraus in das Gewebe getrieben wird.

Im Gegensatz hierzu ist die Steckerbaugruppe einer Elektrodenleitung mit einer so genannten passiven Fixierung nicht drehbar gelagert und gegen eine Drehbewegung fixiert, da diese Fixierung durch am distalen Ende befindliche fest stehende Anker (meist aus Silikon hergestellt) erfolgt, indem sich diese Anker im Trabekelwerk des Ventrikels selbst verhaken.

Die Steckerbaugruppe weist weiter bevorzugt eine entlang der Längsachse befindliche durchgängige Bohrung auf, welche zusammen mit dem durch die gewendelte Zuleitung des Elektrodenkörpers gebildete Lumen eine durchgängige Führung für einen Führungsdraht oder Mandrin bildet.

Um für bestimmte Arten von Elektrodenleitungen das Verdrehen der Stecker zu verhindern, umfasst der Steckerpin weiter bevorzugt eine fest befestigte Eingriffvorrichtung, welche in den Drehschlitz der Schraube eingreift und somit das Verdrehen verhindert. Das Zusammenwirken der Eingriffsvorrichtung mit dem Drehschlitz der Schraube wird dadurch sichergestellt, dass die Position des Steckerpins durch das soeben beschriebene Zusammenwirken zwischen Hohlschraube und Widerlager in proximaler Richtung fixiert ist.

Die Steckerbaugruppe wie alle leitenden Materialien im Anschlussstück sind aus Edelstahl, wie medizinischer Edelstahl oder MP35N, Platin-Iridium-Legierungen, Tantal, Tantal mit einer Beschichtung aus einer Platin-Iridium-Legierung, Elgiloy, oder weiteren implantierbaren und leitfähigen Werkstoffen gefertigt.

Die Aufgabe wird weiterhin durch eine Elektrodenleitung zur kardialen oder neuralen Anwendung mit einer Längsachse, einem proximalen und einem der Therapiestelle zugewandten distalen Ende gelöst, welche am proximalen Ende ein soeben beschriebenes erfindungsgemäßes Anschlussstück umfasst. Die Elektrodenleitung weist weiter einen flexiblen und biegbaren Elektrodenkörper mit einer elektrisch isolierenden Außenhülle auf, welche sich vom proximalen zum distalen Ende des Elektrodenkörpers erstreckt, in der n elektrische Zuleitungen elektrisch isoliert eingebettet sind und eine elektrische Verbindung zwischen den n elektrischen Anschlussleitungen des Anschlussstückes mit n Elektrodenpolen am distalen Ende herstellen. Ebenfalls umfasst ist eine auf der Längsachse des Elektrodenkörpers innerhalb der Außenhülle liegende gewendelte Zuleitung, welche eine elektrische Verbindung zwischen der elektrischen Steckerbaugruppe und einem Elektrodenpol am distalen Ende herstellt. Ein Knickschutz stellt einen Schutz zwischen dem Anschlussstück und dem Elektrodenkörper her, welcher am Anschlussstück befestigt ist.

Vorteilhaft wird die elektrische Verbindung und gleichzeitige mechanische Befestigung zwischen den elektrischen Anschlussleitungen des Anschlussstückes und den eingebetteten elektrischen Zuleitungen des Elektrodenkörpers durch Federelemente aus elektrisch leitfähigem Material hergestellt, welche sowohl an den elektrischen Anschlussleitungen als auch an den elektrischen Zuleitungen befestigt sind, wobei die Federelemente vorzugsweise crimp- und schweißbare Drahtwendeln, besonders bevorzugt Zweifachwendeln sind. So werden die Anforderungen an die Biegewechselfestigkeit erfüllt. Dementsprechend ist das Federelement an den Anschlussleitungen und den Zuleitungen gecrimpt und/oder geschweißt, vorzugsweise durch Laser- oder Widerstandsschweißen.

Vorzugsweise sind die eingebetteten Zuleitungen und die gewendelte Zuleitung Seile, besonders bevorzugt DFT-Seile.

Die Drahtwendeln sind bevorzugt durch Schweißen und Crimpen an den Anschlussleitungen und an den Zuleitungen befestigt. Bevorzugt sind die Zuleitungen, die Federelemente wie auch die Elektrodenpole sowie die leitenden Materialien im Anschlussstück aus Edelstahl, wie medizinischer Edelstahl oder MP35N, Platin-Iridium-Legierungen, Tantal, Tantal mit einer Beschichtung aus einer Platin-Iridium-Legierung, Elgiloy, oder weiteren implantierbaren und leitfähigen Werkstoffen gefertigt.

Der Knickschutz der Elektrodenleitung ist bevorzugt ein Schlauch, auch ausgebildet als "Schlauchtülle", welcher am Anschlag des Anschlussstückes eingerastet und dadurch befestigt ist. Der Anschlag des Anschlussstückes, der vom Stützelement gebildet wird, hat bevorzugt einen Außendurchmesser, der größer ist als die Ringkontakte oder die Isolierabschnitte und so eine nach außen ragende umlaufende Rippe bildet. Der Absatz wirkt mit einer Nut zusammen, die sich im Inneren des Schlauches am distalen Ende befindet. Die Nut ist dabei im Schlauch positioniert, dass der Schlauchabschnitt distal der Nut kürzer als der Abschnitt proximal der Nut ist. Der Schlauch wird - wie weiter unten beschrieben - am Ende des Herstellungsvorganges der Elektrode über das Anschlussstück so geschoben, dass der kurze Schlauchabschnitt in Richtung Anschlussstück liegt. Die Nut rastet bei richtiger Positionierung am Anschlag ein, so dass der proximale lange Schlauchabschnitt über der Anbindungsstelle des Anschlussstückes am Elektrodenkörper liegt und so einen verrutschfreien Knickschutz bildet.

Weiter bevorzugt ist der Schlauch zumindest abschnittsweise als Gewebeschlauch ausgebildet. Als Gewebeschlauch wird erfindungsgemäß ein Schlauch verstanden, in dem ein Flächengebilde aus sich kreuzenden, bevorzugt aus sich im Wesentlichen rechtwinklig kreuzenden, und miteinander verwebten Fäden eingebettet ist. Die Fäden können dabei aus Polyester, bevorzugt das von der Firma Du Pont vertriebene "Dacron", oder aus Polytetrafluorethylen, bevorzugt das von der Firma Du Pont vertriebene "Teflon", hergestellt sein.

Weiterhin wird die Erfindung durch ein Herstellungsverfahren für ein oben beschriebenes Anschlussstück beschrieben, welches folgende Ablaufschritte umfasst:
- Anschweißen der elektrischen Anschlussleitungen an die elektrisch leitenden Kontakte,
- Aufschieben der elektrisch leitenden Kontakte abwechselnd mit den Isolationsabschnitte auf das Stützelement bis zum Anschlag und Ausrichten je einer elektrischen Anschlussleitung in eine der Aussparungen,
- Ausfüllen des durch die elektrisch leitenden Kontakte und die Isolationsabschnitten einerseits und das Stützelement andererseits umgrenzten Innenraums mit einer Spritzmasse, und
- Aushärten der Spritzmasse.

Vorteilig ist, dass dieses Verfahren eine Vorfertigung des Anschlusses ermöglicht, ohne die komplette Elektrodenleitung herzustellen. Dadurch kann ein elektrischer Funktionstest des Anschlussstückes erfolgen und fehlerhaft produzierte Anschlussstücke identifizieren, bevor die komplette Elektrodenleitung hergestellt ist. Dies spart Arbeitszeit, Materialien und damit Kosten.

Weiterhin wird eine erfindungsgemäße Elektrodenleitung durch folgendes Verfahren hergestellt:
- Verbinden der Kontaktstiftes mit der gewendelten Zuleitung,
- Aufschieben des wie oben beschriebenen vormontierten Anschlussstückes auf den Kontaktstift,
- Befestigen des Kontaktstiftes an das Anschlussstück und Anschweißen des Steckerpins an den Kontaktstift,
- Crimpen auf und/oder Schweißen der Federelemente an die eingebetteten Zuleitungen des Elektrodenkörpers,
- Aufschieben des flexiblen Elektrodenkörpers auf die gewendelte Zuleitung,
- Aufschieben und Anschweißen an und/oder Crimpen des Federelementes auf die elektrischen Anschlussleitungen des Anschlussstückes, und
- Aufschieben des Knickschutzes und Einrasten des Knickschutzes am Anschlag.

Vorzugsweise erfolgt das Verbinden des Kontaktstiftes mit der gewendelten Zuleitung des Elektrodenkörpers durch einen Crimp- und/oder einen Schweißvorgang und das Befestigen des Kontaktstiftes an das Anschlussstück durch Einschrauben der Hohlschraube.

Das Dokument US-A-2005/221671 offenbart den nächstliegenden Stand der Technik.

Im Anschluss wird das Anschlussstück und die Elektrodenleitung anhand eines Ausführungsbeispieles beschrieben. Es zeigen:
- Fig. 1:: den Abschnitt einer Elektrodenleitung mit einem erfindungsgemäßen Anschlussstück
- Fig. 2:: das erfindungsgemäße Anschlussstück in einer perspektivischen Darstellung
- Fig. 3:: das erfindungsgemäße Anschlussstück in einem Längsschnitt durch die Längsachse
- Fig. 4a/4b/4c:: das erfindungsgemäße Anschlussstück in der Seitenansicht mit den Schnittlinien B-B und C-C
- Fig. 5:: das Stützelement in einer perspektivischen Ansicht

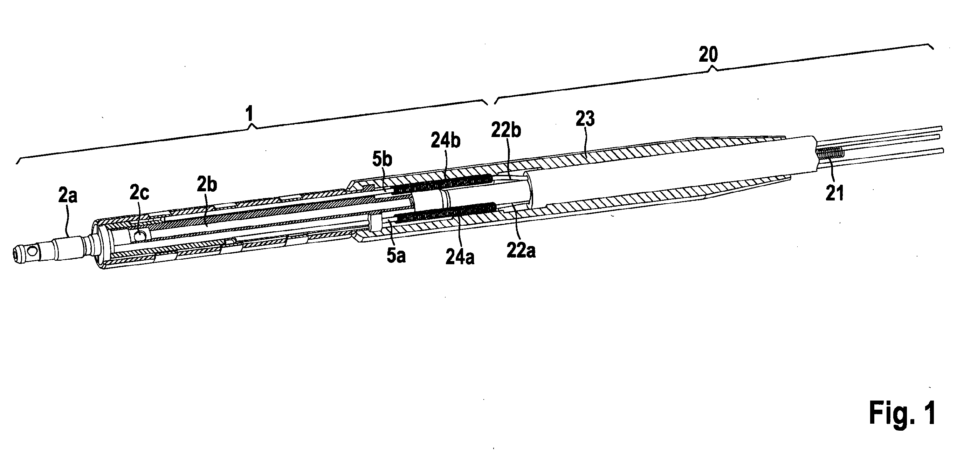

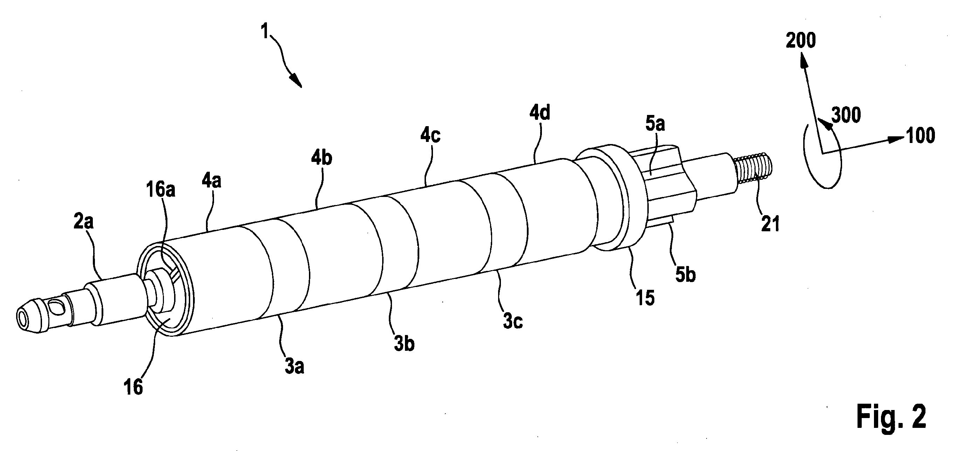

Fig. 1 zeigt einen Abschnitt der erfindungsgemäßen Elektrodenleitung. Die Elektrodenleitung umfasst ein erfindungsgemäßes Anschlussstück 1, welches im folgenden Text weiter beschrieben wird. Weiter umfasst die Elektrodenleitung einen tubularen, flexiblen und biegbaren Elektrodenkörper 20, welcher sich entlang einer Längsachse 100 (wie in Fig. 2 ersichtlich) erstreckt, eine elektrische isolierende biokompatible Außenhülle und ein distales und ein proximales Ende umfasst, wobei das proximale Ende mit dem Anschlussstück 1 verbunden ist. Das distale Ende weist eine nicht dargestellte geeignete Fixierung auf, mit der die Elektrodenleitung am Gewebe, beispielsweise des Herzens, befestigt werden kann, um die Abgabe von elektrischen Impulsen zu ermöglichen. Die Fixierung kann "aktiv" oder auch "passiv" sein, wobei bei einer aktiven Fixierung am distalen eine Befestigungswendel vorgesehen ist, welche aktiv über eine Drehbewegung befestigt werden muss, während eine passive Fixierung fest stehende Verankerungen meist aus Silikon aufweisen, welche sich im Gewebe verhaken.

Der Elektrodenkörper 20 umfasst weiterhin eine gewendelte Zuleitung 21, welche auf der Längsachse 100 liegt. Diese Zuleitung 21 bildet dabei eine Aussparung aus, welche sich ebenfalls entlang der Längsachse 100 erstreckt und ausgestaltet ist, einen Führungsdraht oder andere temporäre Führungsmittel aufzunehmen. Bei einer aktiv fixierbaren Elektrode ist diese elektrische Zuleitung drehbar gelagert und mit der Befestigungswendel am distalen Ende fest verbunden. So kann durch eine Drehbewegung der Zuleitung 21 die Befestigungswendel aktiviert und im Gewebe eingeschraubt werden.

Weiterhin umfasst der Elektrodenkörper mindestens eine Zuleitung 22a oder 22b, welche in der Außenhülle eingebettet sind und so sowohl nach außen als auch bezüglich der gewendelten Zuleitung 21 isoliert sind.

Sowohl die gewendelte Zuleitung 21 als auch die mindestens eine eingebettete Zuleitung 22a oder 22b erstrecken sich entlang der Längsachse 100 vom proximalen zum distalen Ende, sind elektrisch leitend und am proximalen Ende mit dem Anschlussstück 1 und am anderen distalen Ende mit jeweils einem nicht dargestellten Elektrodenpol verbunden. Im Falle der gewendelten Zuleitung 21 kann der Elektrodenpol die Befestigungswendel sein.

Im Übergangsbereich zwischen Anschlussstück 1 und Elektrodenkörper 20 befindet sich ein Knickschutz 23 zum Schutz der Verbindung zwischen Anschlussstück und Elektrodenkörper.

Zur sicheren, elastischen elektrischen Anbindung der eingebetteten Zuleitungen 22a und 22b sind jeder dieser eingebetteten Zuleitungen 22a und 22b Federelemente 24a und 24b zugeordnet. Vorzugsweise kann es sich dabei um Schraubenfedern mit einem Hohlraum handeln, welche über die Zuleitungen 22a und 22b geschoben werden. Dabei stehen die Federelemente 24a und 24b in proximaler Richtung über das Ende der Zuleitungen 22a und 22b heraus, so dass auf dieser Seite ebenfalls elektrische Anschlussleitungen aus der distalen Richtung eingeschoben und befestigt sind. Durch Schweißen und / oder Crimpen sind die zugehörigen Zuleitungen 22a und 22b mit dem jeweiligen Federelement 24a und 24b und das Federelement wiederum mit der jeweiligen Anschlussleitung 5a und 5b fest elektrisch leitend verbunden. So wird sichergestellt, dass die Verbindungsstelle zwischen dem Anschlussstück 1 und dem Elektrodenkörper 20 den Anforderungen an die Biegewechselfestigkeit genügt.

Die Federelemente 24a und 24b liegen unterhalb des Knickschutzes 23 und werden durch diesen geschützt.

Das Herstellungsverfahren einer solchen Elektrodenleitung umfasst die folgenden Schritte:
- Verbinden der Kontaktstiftes 2b mit der gewendelten Zuleitung 21,
- Aufschieben des vormontierten Anschlussstückes 1 auf den Kontaktstift 2b,
- Befestigen des Kontaktstiftes 2b an das Anschlussstück und Anschweißen des Steckerpins 2a an den Kontaktstift 2b,
- Crimpen auf und/oder Schweißen der Federelemente 24a und 24b an die eingebetteten Zuleitungen 22a und 22b des Elektrodenkörpers,
- Aufschieben des flexiblen Elektrodenkörpers auf die gewendelte Zuleitung,
- Aufschieben und Schweißen an und/oder Crimpen des Federelementes 24a und 24b auf die elektrischen Anschlussleitungen 5a bis 5c, und
- Aufschieben des Knickschutzes 23 und Einrasten des Knickschutzes am Anschlag 15.

Das Verbinden des Kontaktstiftes 2b mit der gewendelten Zuleitung des Elektrodenkörpers erfolgt durch einen Crimp- und/oder Schweißvorgang, und das Befestigen des Kontaktstiftes an das Anschlussstück durch Einschrauben der Hohlschraube 16.

Im Folgenden wird das erfindungsgemäße Anschlussstück beschrieben.

Fig. 2 veranschaulicht ein freigeschnittenes und eigenständiges vierpoliges IS-4-Anschlussstück 1, wie es sich in einer Außenansicht darstellt. Zu sehen ist ein IS-4-Steckerpin 2a, der am proximalen Ende des Anschlussstückes proximal aus dem Anschlussstück herausragt. Auf der Außenseite des Anschlussstückes sind drei elektrisch leitende Kontakte 3a, 3b und 3c in Form von Ringkontakten und abwechselnd Isolationsabschnitte 4a bis 4d dargestellt. Die elektrisch leitenden Kontakte und die Isolationsabschnitte haben denselben Außendurchmesser. Weiterhin ist ein Absatz 15 zur Befestigung eines hier nicht dargestellten Knickschutzes 23 sichtbar. Zur deutlicheren Veranschaulichung der Wirkungsweisen und der Bauteile werden hier die Begriffe der Längsachse oder Längsrichtung 100, entlang das Anschlussstück 1 aufgebaut ist, der im rechten Winkel zur Längsachse liegenden Radial oder Radialrichtung 200, entlang der ein Verschieben der Komponenten durch das Stützelement verhindert wird, und der Umfangrichtung 300, die sich um die Längsachse dreht und entlang der sich die elektrischen Kontaktflächen befinden, eingeführt.

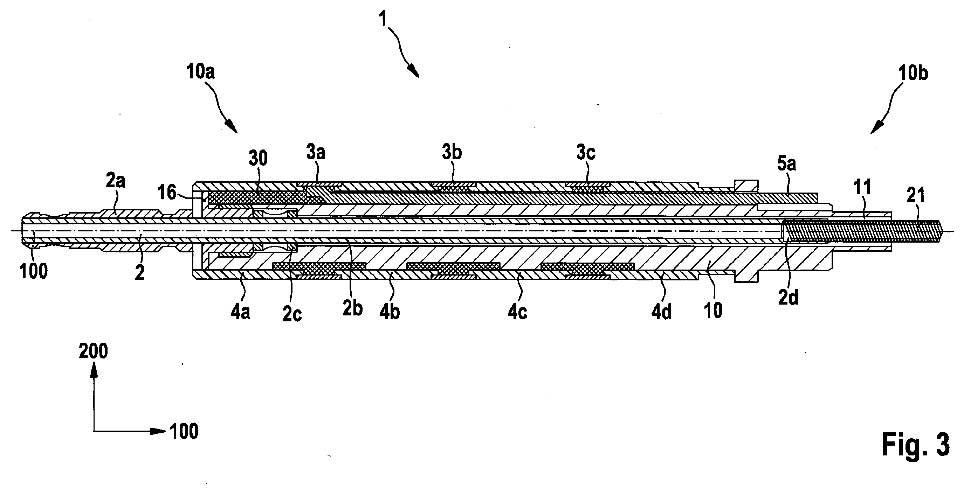

Fig. 3 zeigt das Anschlussstück 1 für eine vierpolige Elektrodenleitung in einer Schnittdarstellung entlang der Längsachse 100. Das Anschlussstück ist vom Elektrodenkörper freigeschnitten und im komplett montierten Zustand als eigenständige Einheit dargestellt.

Das Anschlussstück 1 weist eine lang gestreckte Form entlang einer Längsachse 100 auf und bildet ein proximales Ende 10a und ein distales Ende 10b aus. Eine Steckerbaugruppe 2 erstreckt sich durch fast das komplette Anschlussstück und ragt proximal aus dem proximalen Ende 10a mit einem Steckerpin 2a - in dieser Ausführung ist der Steckerpin als genormter IS-4-Pin ausgestaltet - aus dem Anschlussstück heraus. Die Steckerbaugruppe 2 umfasst weiterhin einen Kontaktstift 2b, auf dessen proximalen Ende der Steckerpin 2a mittels Schweißen fest befestigt ist. Am distalen Ende des Kontaktstiftes kann eine gewendelte Zuleitung 21 des Elektrodenkörpers fest durch Crimpen und/oder Schweißen verbunden werden. Dazu weist der Kontaktstift eine Vertiefung 2d auf, in die die gewendelte Zuleitung eingebracht und gecrimpt oder geschweißt werden kann. Vorteilhaft liegt die Vertiefung zum Schutz innerhalb des Anschlussstückes. Der Pin ist zunächst frei drehbar um die Längsachse 100 gelagert.

Weiterhin weist das Anschlussstück elektrisch leitende Kontakte 3a, 3b und 3c, deren Außenfläche eine elektrische Verbindung zu korrespondierenden elektrisch leitenden Innenkontakten im Anschlussgehäuse (auch Header genannt) eines elektrischen Implantats herstellen. So werden die Signale aus oder zu dem Implantat an die Elektrodenpole einer Elektrodenleitung weitergeleitet. Die elektrisch leitenden Kontakte 3a, 3b und 3c werden durch Isolationsabschnitte 4a, 4b, 4c und 4d gegeneinander elektrisch isoliert. Das heißt, die elektrisch leitenden Kontakte und die Isolationsabschnitte wechseln sich entlang der Längsachse 100 ab, wobei die beiden zwischen den Kontakten liegenden Isolationsabschnitte 4b und 4c identisch sind, der Isolationsabschnitt 4a eine proximale Abschlusskante aufweist, um das Anschlussstück proximal abzuschließen, und der Isolationsabschnitt 4d eine außen liegende umlaufende Vertiefung aufweist, die dazu ausgestaltet ist, mit einem nicht dargestellten Knickschutz 23 zusammenzuwirken. Jeder der Isolationsabschnitte weist auf der den elektrischen Kontakten zugewandten Seite Absätze auf, die in ihrer Tiefe der Höhe der elektrischen Kontakte entsprechen. Die elektrischen Kontakte kommen auf diesen Absätzen zum Liegen und werden so durch die Isolationsabschnitte in Längsrichtung 100 fixiert, während die Isolationsabschnitte wiederum durch das Stützelement in Radialrichtung 200 und durch den Anschlag 15 in Längsrichtung 100 fixiert werden. Weiterhin wird so sichergestellt, dass die elektrischen Kontakte 3a, 3b, 3c und die Isolationsabschnitte 4a, 4b, 4c und 4d normgerecht denselben Außendurchmesser aufweisen.

Die Isolationsabschnitte 4a bis 4d, die ebenfalls eine Ringform aufweisen, grenzen auf ihrer in Radialrichtung weiter innen liegenden Innenfläche an ein Stützelement 10 an. Dadurch wird verhindert, dass die Isolationsabschnitte in Radialrichtung verschiebbar sind.
Das Stützelement 10 weist einen im Wesentlichen kreisrunden Außenquerschnitt und eine Längsachse entlang der Längsrichtung 100 des Anschlussstückes auf. Dabei bildet das Stützelement die komplette Länge des Anschlussstückes ohne den Steckerpin 2a aus. Auf der Längsachse befindet sich eine durchgängige Bohrung 11, welche dazu ausgestaltet ist, die Steckerbaugruppe 2 aufzunehmen und drehbar zu lagern. Die Bohrung 11 weist am proximalen Ende 10a des Stützelementes eine Aussparung und ein Innengewinde auf, in das ein Widerlager 2c, das fest mit dem Kontaktstift 2b der Steckerbaugruppe 2 verbunden ist, eingeführt werden kann. Proximal des Widerlagers 2c wird eine Hohlschraube 16 eingeführt, die eine Durchgangsbohrung mit einem Durchmesser hat, der dem Außendurchmesser des Kontaktstiftes 2b entspricht. Das Widerlager kann in proximaler Richtung nicht bewegt werden, weil es durch die Hohlschraube gesperrt wird. So wirkt also das Widerlager 2c mit der Hohlschraube zusammen und verhindert, dass der Kontaktstift 2b und damit die Steckerbaugruppe 2 proximal aus dem Anschlussstück 1 herausgezogen werden kann. Mit anderen Worten: Das axiale Spiel kann so minimiert werden, dass eine axiale Verschiebung bei eingeschraubter Hohlschraube nicht mehr möglich ist. Andererseits bewirkt die Hohlschraube 16 auch, dass der Steckerpin 2a, der ebenfalls einen Außendurchmesser aufweist, der größer als der Innendurchmesser der Hohlschraube ist, dass die Steckerbaugruppe 2 auch nicht in distaler Richtung entlang der Längsachse verschiebbar ist. Durch diese Anordnung bleibt eine um die Längsachse drehbare Lagerung erhalten. Um diese Drehbarkeit der Steckerbaugruppe zu verhindern, kann beispielsweise eine Eingriffsvorrichtung am Steckerpin 2a vorgesehen werden, die in den Drehschlitz der Hohlschraube 16 eingreift. Damit wird die Drehbarkeit verhindert.

In Fig. 3 ist weiterhin zu sehen, dass elektrisch leitende Anschlussleitungen jeweils einem elektrisch leitenden Kontakt 3a, 3b oder 3c zugeordnet sind. Zu sehen ist die Anschlussleitung 5a, welche mit dem elektrisch leitenden Kontakt 3a elektrisch leitend durch eine Schweiß- oder Lötverbindung verbunden ist. Die Leitung wird parallel zur Längsachse in distale Richtung geführt, um dort elektrisch an Zuleitungen eines Elektrodenkörpers angebunden zu werden. Die Zuleitungen der anderen elektrisch leitenden Kontakte werden entsprechend geführt und befinden sich um die Längsachse verteilt in Aussparungen.

Wie weiter aus Fig. 3 ersichtlich, bilden das Stützelement 10 einerseits und die elektrisch leitenden Kontakte 3a, 3b, 3c und die Isolationsabschnitte 4a bis 4d andererseits einen Innenraum, der mit Spritzmasse 30 ausgefüllt ist. Die Spritzmasse besteht dabei aus einem einzigen Material, welches vorzugsweise dem Material entspricht, aus dem das Stützelement 10 hergestellt ist. Dies ist vorzugsweise PEKK. Durch diesen ausgefüllten Innenraum in Zusammenwirkung mit den Isolationsabschnitten erfüllt dieses Anschlussstück 1 die Anforderungen an die Hochspannungs- und Überschlagfestigkeit.

Das Anschlussstück wird durch einen Prozess hergestellt, der die folgenden Schritte umfasst:
- Anschweißen der elektrischen Anschlussleitungen 5a bis 5c an die elektrisch leitenden Kontakte 3a bis 3c,
- Aufschieben der elektrisch leitenden Kontakte abwechselnd mit den Isolationsabschnitten 4a bis 4d auf das Stützelement 10 bis zum Anschlag 15 und Ausrichten je einer elektrischen Anschlussleitung in eine der Aussparungen 13a bis 13c,
- Ausfüllen des durch die elektrisch leitenden Kontakte 3a bis 3c und die Isolationsabschnitten 4a bis 4d einerseits und das Stützelement 10 andererseits umgrenzten Innenraums mit einer Spritzmasse, und
- Aushärten der Spritzmasse.

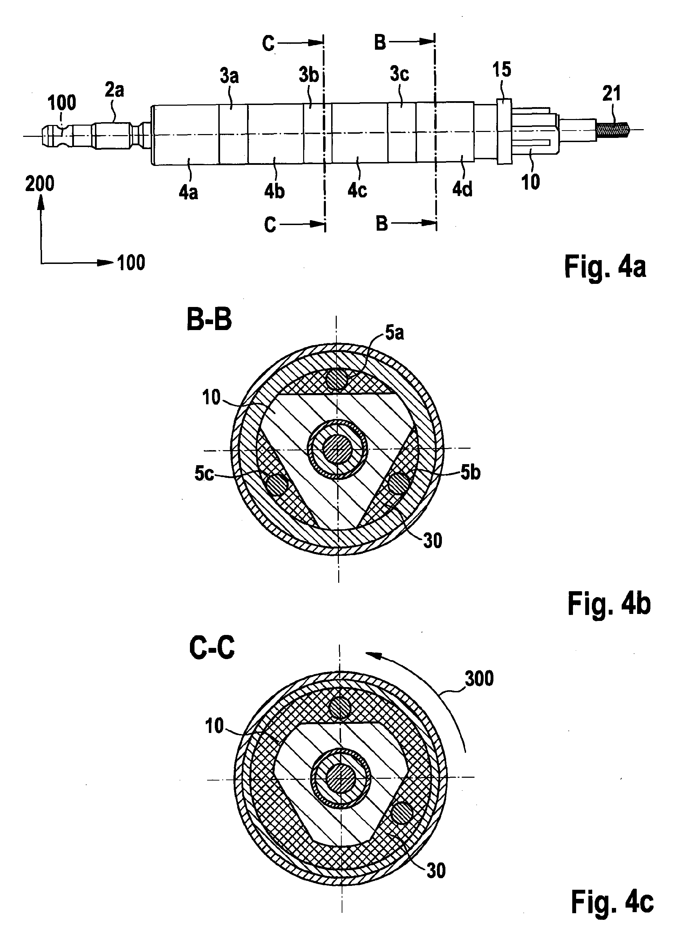

Die Fig. 4a bis 4c zeigen ein so hergestelltes Anschlussstück in der Seitenansicht mit den elektrisch leitenden Kontakten 3a bis 3c, den Isolationsabschnitten 4a bis 4d, Teilen des Stützelements 10 mit dem Anschlag 15. In diesem Ausführungsbeispiel ist ein Steckerpin 2a an das proximale Ende und eine wendelförmige Zuleitung 21 an das distale Ende des nicht sichtbaren innen liegenden Kontaktstiftes angeschweißt und/oder aufgecrimpt. Exemplarisch werden zwei Schnitte entlang der Schnittkanten B-B (Fig. 4b) und C-C (Fig. 4c) gezeigt. Aus den Schnitten ist der Innenraum zwischen den elektrisch leitenden Kontakten und den Isolationsabschnitten einerseits und dem Stützelement andererseits ersichtlich, das mit einer Spritzmasse 30 ausgefüllt ist. Die in diesem Innenraum verlaufenden Anschlussleitungen 5a bis 5c liegen auf einer Seite auf dem Stützelement 10 auf und sind auf den anderen Seiten mit Spritzmasse 30 umgossen. So entsteht eine optimale Isolierung, die Hochspannungs- und Überschlagfestigkeit gewährleistet. Zusätzlich können die einzelnen Zuleitungen mit einer zusätzlichen Isolierschicht umhüllt werden, um den genannten Effekt weiter zu optimieren. Weiterhin ist zu sehen, dass die Isolationsabschnitte eine wesentlich größere Ausdehnung in Radialrichtung 200 aufweisen als die Ringkontakte. Die beeinflusst die Hochspannungs- und Überschlagfestigkeit weiter positiv.

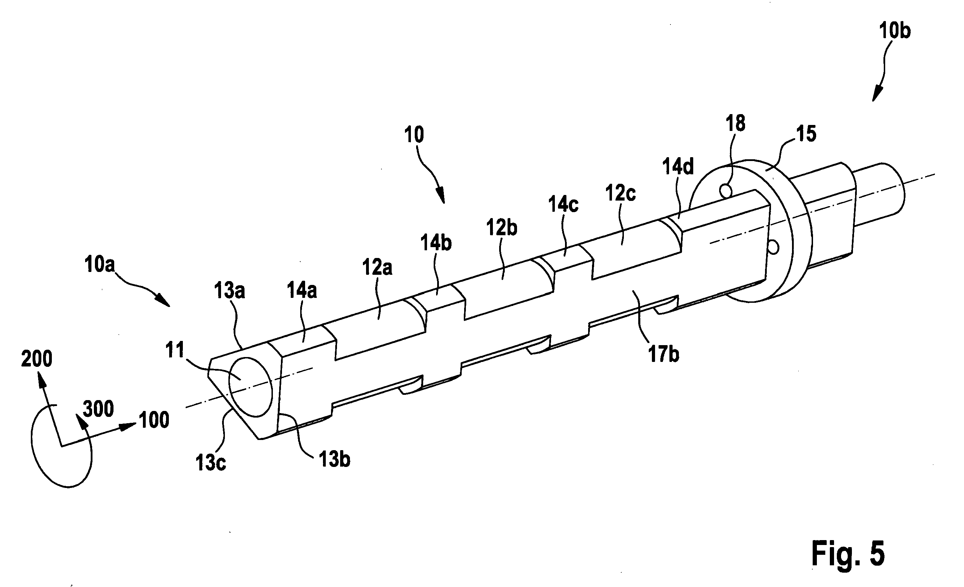

Fig. 5 zeigt das erfindungsgemäße Stützelement 10 mit einem im Wesentlichen kreisrunden Außenquerschnitt und mit einer Längsachse entlang der Längsrichtung 100. Das Stützelement ist ausgebildet, die nicht gezeigten Isolationsabschnitte zu tragen und in Radialrichtung 200 zu fixieren. Weiterhin weist es eine auf seiner Längsachse befindlichen durchgehende Bohrung 11 auf, welche den elektrisch leitenden Kontaktstift 2b und/oder eine gewendelte Zuleitung 21 des Elektrodenkörpers aufnimmt. Das Stützelement weist an seinem proximalen Ende 10a eine Aussparung und ein Innengewinde auf, wobei die Aussparung einen größeren Durchmesser als die Bohrung 11 aufweist. Diese Aussparung ist wie weiter oben beschrieben zur Aufnahme eines Widerlagers und einer Hohlschraube ausgebildet.

Das dargestellte Stützelement, welches für ein Anschlussstück mit drei elektrisch leitenden Kontakten und einen Steckerpin vorgesehen ist, weist drei umlaufende Nuten 12a bis 12c auf. Diese dienen einerseits dazu, die Spritzmasse aufzunehmen und zu verteilen. Andererseits dienen sie als Aussparung für den Schweiß- oder Lötkontakt zwischen den elektrisch leitenden Kontakten und den Anschlussleitungen. Die Ringkontakte kommen also in radialer Richtung über den Nuten zum Liegen, während die Isolationsabschnitte mittig auf den Rippen 14a bis 14d liegen. Der Außendurchmesser der Rippen kann derart dimensioniert sein, dass eine Passung zum Innendurchmesser der Isolationsabschnitte entsteht. Dies bewirkt einen stabileren Sitz der Isolationsabschnitte und der von ihnen fixierten elektrisch leitenden Kontakte und dient der Fixierung in Radialrichtung 200. Die Rippen 14a bis 14d weisen bevorzugt eine Breite von 1 bis 6 mm und besonders bevorzugt zwischen 1,5 und 5,25 mm auf.

Weiterhin sind drei sich vom proximalen 10a bis zum distalen Ende 10b parallel der Längsachse 100 erstreckende Aussparungen 13a, 13b, 13c vorhanden. In diesen Aussparungen verlaufen die nicht dargestellten Anschlussleitungen von dem jeweiligen elektrisch leitenden Kontakt, mit dem die Anschlussleitungen verbunden sind, mindestens bis zum distalen Ende 10b, wobei die Anschlussleitungen distal über das distale Ende des Stützelements herausragen können, um besser mit den Zuleitungen des Elektrodenkörpers montiert zu werden. Die Anzahl der Nuten und die Anzahl der Aussparungen entsprechen dabei der Anzahl der elektrisch leitenden Kontakte.

Durch die Anzahl der elektrisch leitenden Kontakte wird dabei auch maßgeblich die Form der entlang der Längsachse verlaufenden Aussparungen beeinflusst. Bei einer geringeren Anzahl an elektrisch leitenden Kontakten sind die Begrenzungen der Aussparungen in Richtung Längsachse von flächiger Natur. Es bilden sich also "Sekantenflächen", die bei Zylindern entstehen, wenn die Grundfläche des Zylinders durch Sekanten zugeschnitten werden. In Fig. 5 ist die "Sekantenfläche" 17b zu sehen, die im Wesentlichen eine identische Gestalt zu den beiden anderen nicht sichtbaren Flächen 17a und 17c aufweist. Je größer die Anzahl an Aussparungen wird, desto mehr muss die Fläche durch eine Komponente in Radialrichtung ergänzt werden, so dass u- oder v-förmige Begrenzungen in Richtung der Längsachse entstehen. Nur dadurch kann sichergestellt werden, dass die Anschlussleitungen sicher geführt werden können. Der Querschnitt des Stützelements 10 nimmt also mit zunehmender Anzahl an elektrisch leitenden Kontakten eine "sternförmige Gestalt" oder auch die Gestalt eines N-Ecks an, wobei die sich bildenden Kanten und Flächen von der Längsachse maximal von einem Hüllkreis als Außendurchmesser begrenzt wird.

In der in Fig. 5 vorliegenden Ausgestaltung werden die Außenflächen der Rippen 14a bis 14d durch Abschnitte des Hüllkreises gebildet und bilden gleichzeitig die Passung für die nicht dargestellten Isolationsabschnitte.

Das Stützelement 10 weist am oder in der Nähe des distalen Endes 10b einen umlaufenden Anschlag 15 auf, der einen Außendurchmesser hat, der größer ist als der Hüllkreis des Außenquerschnittes des restlichen Stützelementes 10. Dieser Anschlag dient zum Einen zur Montageerleichterung bei der Vormontage des Anschlussstückes 1. So kann der nicht dargestellte Isolationsabschnitt (in Fig. 2 mit dem Bezugszeichen 4d dargestellt) einfach vom proximalen Ende 10a auf das Stützelement aufgeschoben werden, bis er am Anschlag 15 zu liegen kommt. Anschließend werden die elektrisch leitenden Kontakte 3a bis 3c (dargestellt in Fig. 2) - nachdem sie mit der jeweiligen Anschlussleitung 5a bis 5c (dargestellt in Fig. 2) fest verbunden wurden - abwechselnd mit den restlichen Isolationsabschnitten 4a bis 4c (dargestellt in Fig. 2) aufgeschoben, so dass die elektrisch leitenden Kontakte auf den Absätzen der Isolationsabschnitte zum Liegen kommen. Durch diese Montageweise werden die elektrisch leitenden Kontakte an der dafür vorgesehenen Stelle montiert, ohne dass Toleranzen vorgesehen werden müssen. Die Anschlussleitungen werden während des Aufschiebens in die jeweiligen Aussparungen 13a bis 13c im sich bildenden Innenraum in Richtung des distalen Endes 10b eingeführt und durch Bohrungen 18, die distal zu den Aussparungen 13a bis 13c liegen, durch den Anschlag 15 geführt. Es ist auch vorstellbar, anstatt diese Aussparung in Längsrichtung durch den Anschlag 15 durch eine Bohrung 18 zu bilden, durch eine nach außen offene Aussparung zu bilden, die in Verlängerung hinter den Aussparungen13a bis 13c liegen. Dies hat den Vorteil, dass die Anschlussleitungen und damit das Anschlussstück noch leichter montiert werden können.

Andererseits wirkt der Anschlag 15 mit einer Nut am Knickschutz 23 zusammen, die sich im Inneren des Schlauches am distalen Ende befindet, das heißt, der Schlauchabschnitt distal der Nut ist kürzer als der Abschnitt proximal der Nut. Der Schlauch wird wie weiter oben beschrieben, am Ende des Herstellungsvorganges der Elektrodenleitung so über das Anschlussstück geschoben, dass der kurze Schlauchabschnitt in Richtung Anschlussstück liegt. Die Nut rastet bei richtiger Positionierung am Anschlag 15 ein, so dass der proximale lange Schlauchabschnitt über der Anbindungsstelle des Anschlussstückes am Elektrodenkörper liegt und so einen verrutschfreien Knickschutz 23 bildet, der über den Federelementen 24a und 24b liegen.

## Patentansprüche

1. Anschlussstück (1) ausgebildet als eigenständige Einheit zum Verbinden mit einer Elektrodenleitung und zum Anschluss an ein elektrisches Implantat, wobei das Anschlussstück eine lang gestreckte Form in einer Längsrichtung (100) und ein proximales und ein distales Ende (10a, 10b) aufweist und folgendes umfasst:
- eine elektrisch leitende Steckerbaugruppe (2), die in Längsrichtung (100) proximal aus dem proximalen Ende des Anschlussstückes ragt,
- mehrere ringförmige, elektrisch leitende Kontakte (3a, 3b, 3c) gleichen Außendurchmessers mit in Längsrichtung dazwischen angeordneten Isolationsabschnitten (4a, 4b, 4c, 4d) desselben Außendurchmessers, wobei die Isolationsabschnitte die elektrisch leitenden Kontakte derart fixieren, dass ihre Lage in Längsrichtung (100) unveränderlich sind,
- mehrere elektrische Anschlussleitungen (5a, 5b, 5c), die eine elektrische Verbindung mit jeweils einer korrespondierenden Zuleitung des Elektrodenkörpers am distalen Ende des Anschlussstückes verbindet, und
- ein Stützelement (10) mit einem im Wesentlichen kreisrunden Außenquerschnitt und mit einer Längsachse entlang der Längsrichtung, welches ausgebildet ist, die Isolationsabschnitte zu tragen und in Radialrichtung (200) zu fixieren, und welches eine auf seiner Längsachse befindliche durchgehende Bohrung (11) aufweist, welche die Steckerbaugruppe (2) und/oder eine Zuleitung eines Elektrodenkörpers aufnimmt,
wobei
das Stützelement n in Längsrichtung beabstandete umlaufende Nuten (12a, 12b, 12c), welche ausgebildet sind, eine Spritzmasse aufzunehmen, und n sich vom proximalen bis zum distalen Ende parallel der Längsachse erstreckende Aussparungen (13a, 13b, 13c) aufweist, um Anschlussleitungen (5a, 5b, 5c) von den elektrisch leitenden Kontakten (3a, 3b, 3c) zum distalen Ende zu führen, wobei n der Anzahl der elektrisch leitenden Kontakte entspricht.

2. Anschlussstück nach Anspruch 1, **dadurch gekennzeichnet, dass** die elektrisch leitenden Kontakte (3a, 3b, 3c) und die Isolationsabschnitte (4a, 4b, 4c, 4d) einerseits und das Stützelement (10) andererseits einen Innenraum einschließen, in den die Spritzmasse nach dem Zusammenfügen der elektrisch leitenden Kontakte, der Isolationsabschnitte und des Stützelements durch Spritzgießen eingebracht ist und so die elektrisch leitenden Kontakte, Isolationsabschnitte und das Stützelement fest miteinander verbindet.

3. Anschlussstück nach Anspruch 2, **dadurch gekennzeichnet, dass** der Innenraum durch umlaufenden Nuten (12a, 12b, 12c) und die sich vom proximalen bis zum distalen Ende parallel der Längsachse erstreckende Aussparungen (13a, 13b, 13c) des Stützelements (10) gebildet ist.

4. Anschlussstück nach einem der Ansprüche 1 bis 3, **dadurch gekennzeichnet, dass** die umlaufenden Nuten (12a, 12b, 12c) so gegeneinander beabstandet sind, dass die in Längsrichtung vor und hinter den Nuten liegenden umlaufenden Rippen (14a, 14b, 14c, 14d) die Isolationsabschnitte (4a, 4b, 4c, 4d) tragen und einen Außendurchmesser aufweisen, der einen passgenauen Sitz für den jeweiligen Isolationsabschnitt ermöglicht.

5. Anschlussstück nach Anspruch 4, **dadurch gekennzeichnet, dass** die Rippen (14a, 14b, 14c, 14d) eine Breite von 1 bis 6 mm, vorzugsweise zwischen 1,5 und 5,25 mm aufweisen.

6. Anschlussstück nach Anspruch 1 oder 3, **dadurch gekennzeichnet, dass** die entlang der Längsachse verlaufenden Aussparungen (13a, 13b, 13c) radial nach außen offen sind, so dass leicht die elektrischen Anschlussleitungen eingebracht werden können..

7. Anschlussstück nach Anspruch 6, **dadurch gekennzeichnet, dass** die Begrenzungen der Aussparungen (13a, 13b, 13c) in Richtung Längsachse durch Sekantenflächen (17a, 17b, 17c) begrenzt sind, so dass das Stützelement (10) einen polyedrischen Querschnitt, vorzugsweise mit einem gleichseitigen N-Eck mit einem Hüllkreis als Querschnitt aufweist.

8. Anschlussstück nach einem der Ansprüche 1 bis 7, **dadurch gekennzeichnet, dass** das Stützelement (10) einen umlaufenden Anschlag (15) am distalen Ende oder in dessen Nähe aufweist, um die exakte Montage der elektrischen Kontakte und Isolationsabschnitte zu ermöglichen.

9. Anschlussstück nach einem der Ansprüche 1 bis 8, **dadurch gekennzeichnet, dass** das Stützelement (10) aus einem Polymer, vorzugsweise aus PEEK (Poly Ether Ether Keton), aus PEKK (Poly Ether Keton Keton oder aus POM (Polyoxymethylen), weiter bevorzugt aus Keramiken, hergestellt ist und die Spritzmasse aus einem einzigen Material, vorzugsweise Polyurethan, PEEK (Poly Ether Ether Keton), PEKK (Poly Ether Keton Keton) oder aus POM (Polyoxymethylen), weiter bevorzugt aus Keramiken oder besonders bevorzugt aus denselben Materialien wie das Stützelement (10) besteht.

10. Anschlussstück nach Anspruch 1, **dadurch gekennzeichnet, dass** die Steckerbaugruppe (2) einen Steckerpin (2a) zum Kontaktieren mit dem elektrischen Implantat und einen Kontaktstift (2b) zum Herstellen eines Kontaktes mit einer Zuleitung des Elektrodenkörper aufweist, wobei der Kontaktstift bevorzugt ein Widerlager (2c) aufweist, welches mit einer im Stützelement (10) eingeschraubten Hohlschraube (16) so zusammenwirkt, dass die Steckerbaugruppe (2) in Längsrichtung (100) im Stützelement fixiert ist.

11. Elektrodenleitung zur kardialen oder neuralen Anwendung mit einer Längsachse, einem proximalen und einem der Therapiestelle zugewandten distalen Ende, welche umfasst:
- ein Anschlussstück nach einem der Ansprüche 1 bis 10 am proximalen Ende,
- einen flexiblen und biegbaren Elektrodenkörper mit einer elektrisch isolierenden Außenhülle, welche sich vom proximalen zum distalen Ende des Elektrodenkörpers erstreckt und in der n elektrische Zuleitungen elektrisch isoliert eingebettet sind und eine elektrische Verbindung zwischen den n elektrischen Anschlussleitungen (5a, 5b, 5c) mit n Elektrodenpolen am distalen Ende herstellen,
- mindestens eine auf der Längsachse innerhalb der Außenhülle liegende gewendelte Zuleitung, welche eine elektrische Verbindung zwischen der elektrischen Steckerbaugruppe (2) und einem Elektrodenpol am distalen Ende herstellt, und
- einen Knickschutz (23), der am Anschlussstück befestigt ist.

12. Elektrodenleitung nach Anspruch 11, **dadurch gekennzeichnet, dass** die elektrische Verbindung und die gleichzeitige mechanische Befestigung zwischen den elektrischen Anschlussleitungen (5a, 5b, 5c) und den eingebetteten elektrischen Zuleitungen durch Federelemente aus elektrisch leitfähigem Material hergestellt wird, welche sowohl an den elektrischen Anschlussleitungen (5a, 5b, 5c) als auch an den elektrischen Zuleitungen befestigt sind, wobei die Federelemente vorzugsweise crimp- und schweißbare Drahtwendeln, besonders bevorzugt Zweifachwendeln, sind.

13. Elektrodenleitung nach einem der Ansprüche 11 oder 12, **dadurch gekennzeichnet, dass** die eingebetteten Zuleitungen und die gewendelte Zuleitung Seile, besonders bevorzugt DFT-Seile, sind.

14. Elektrodenleitung nach einem der Ansprüche 12 bis 13, **dadurch gekennzeichnet, dass** die Zuleitungen, die Federelemente wie auch die Elektrodenpole sowie die leitenden Materialien im Anschlussstück aus Platin-Iridium-Legierungen, Tantal, Tantal mit einer Beschichtung aus einer Platin-Iridium-Legierung, Elgiloy, oder weiteren implantierbaren und leitfähigen Werkstoffen gefertigt sind.

15. Verfahren zur Herstellung eines Anschlussstückes nach einem der Ansprüche 1 bis 10, welches folgende Schritte umfasst:
- Anschweißen der elektrischen Anschlussleitungen (5a, 5b, 5c) an die elektrisch leitenden Kontakte (3a, 3b, 3c),
- Aufschieben der elektrisch leitenden Kontakte abwechselnd mit den Isolationsabschnitten (4a, 4b, 4c, 4d) auf das Stützelement (10) bis zum Anschlag (15) und Ausrichten je einer elektrischen Anschlussleitung in eine der Aussparungen (13a, 13b, 13c),
- Ausfüllen des durch die elektrisch leitenden Kontakte (3a, 3b, 3c) und die Isolationsabschnitten (4a, 4b, 4c, 4d) einerseits und das Stützelement (10) andererseits umgrenzten Innenraums mit einer Spritzmasse, und
- Aushärten der Spritzmasse.

16. Verfahren zur Herstellung einer Elektrodenleitung nach einem der Ansprüche 11 bis 14, welches folgende Schritte umfasst:
- Verbinden des Kontaktstiftes (2b) mit der gewendelten Zuleitung,
- Aufschieben des nach Anspruch 15 vormontierten Anschlussstückes auf den Kontaktstift (2b),
- Befestigen des Kontaktstiftes (2b) an das Anschlussstück und Anschweißen des Steckerpins (2a) an den Kontaktstift (2b),
- Crimpen auf und/oder Schweißen der Federelemente an die eingebetteten Zuleitungen des Elektrodenkörpers,
- Aufschieben des flexiblen Elektrodenkörpers auf die gewendelte Zuleitung,
- Aufschieben und Schweißen an und/oder Crimpen des Federelementes auf die elektrischen Anschlussleitungen (5a, 5b, 5c) des Anschlussstückes, und
- Aufschieben des Knickschutzes (23) und Einrasten des Knickschutzes am Anschlag (15).

17. Verfahren nach Anspruch 16, **dadurch gekennzeichnet, dass** das Verbinden des Kontaktstiftes (2b) mit der gewendelten Zuleitung des Elektrodenkörpers durch einen Crimp- und/oder einen Schweißvorgang erfolgt und das Befestigen des Kontaktstiftes an das Anschlussstück durch Einschrauben der Hohlschraube (16) erfolgt.

## Claims

1. A connection piece (1) formed as an independent unit for connection to an electrode line and for connection to an electric implant, wherein the connection piece has an elongate form in a longitudinal direction (100) and has a proximal end and a distal end (10a, 10b) and comprises the following:
- an electrically conducting plug module (2), which protrudes proximally from the proximal end of the connection piece,
- a plurality of annular, electrically conducting contacts (3a, 3b, 3c) having the same outer diameter with insulation portions (4a, 4b, 4c, 4d) that are arranged in between them in the longitudinal direction and have the same outer diameter, wherein the insulation portions fix the electrically conducting contacts in such a way that their length in the longitudinal direction (100) is unchangeable,
- a plurality of electric connection lines (5a, 5b, 5c), which connect an electric connection in each case to a corresponding feed line of the electrode body at the distal end of the connection piece, and
- a supporting element (10) with a substantially circular outer cross section and with a longitudinal axis along the longitudinal direction, which is designed to carry the insulation portions and to fix said insulation portions in the radial direction (200), and which has a continuous bore (11) located over its longitudinal axis, said bore receiving the plug module (2) and/or a feed line of an electrode body,
wherein
the support element has n peripheral grooves (12a, 12b, 12c), which are spaced in the longitudinal direction and which are designed to receive an injection compound, and n recesses (13a, 13b, 13c) extending from the proximal end to the distal end, parallel to the longitudinal axis, in order to guide connection lines (5a, 5b, 5c) from the electrically conducting contacts (3a, 3b, 3c) to the distal end, wherein n corresponds to the number of the electrically conducting contacts.

2. The connection piece according to Claim 1, **characterised in that** the electrically conducting contacts (3a, 3b, 3c) and the insulation portions (4a, 4b, 4c) on the one hand and the supporting element (10) on the other hand enclose an interior, into which the injection compound is introduced once the electrically conducting contacts, the insulation portions and the supporting element have been joined together by injection moulding, and thus fixedly interconnecting the electrically conducting contacts, insulation portions and the supporting element with each other.

3. The connection piece according to Claim 2, **characterised in that** the interior is formed by peripheral grooves (12a, 12b, 12c) and the recesses (13a, 13b, 13c) of the supporting element (10) extending from the proximal end to the distal end, parallel to the longitudinal axis.

4. The connection piece according to one of Claims 1 to 3, **characterised in that** the peripheral grooves (12a, 12b, 12c) are spaced in relation to one another such that the peripheral ribs (14a, 14b, 14c, 14d) arranged in front of and behind the grooves in the longitudinal direction carry the insulation portions (4a, 4b, 4c, 4d) and have an outer diameter that enables a seat with an accurate fit for the respective insulation portion.

5. The connection piece according to Claim 4, **characterised in that** the ribs (14a, 14b, 14c, 14d) have a width from 1 to 6 mm, preferably between 1.5 and 5.25 mm.

6. The connection piece according to Claim 1 or 3, **characterised in that** the recesses (13a, 13b, 13c) running along the longitudinal axis are radially outwardly open, such that the electric connection lines can easily be introduced.

7. The connection piece according to Claim 6, **characterised in that** the delimitations of the recesses (13a, 13b, 13c) are delimited in the direction of the longitudinal axis by secant surfaces (17a, 17b, 17c), such that the supporting element (10) has a polyhedral cross section, preferably with a cross-section in the form of an equilateral N-sided polygon with an enveloping circle.

8. The connection piece according to one of Claims 1 to 7, **characterised in that** the supporting element (10) has a peripheral stop (15) at the distal end or in the vicinity thereof in order to enable the exact assembly of the electric contacts and insulation portions.

9. The connection piece according to one of Claims 1 to 8, **characterised in that** the supporting element (10) is produced from a polymer, preferably from PEEK (polyether ether ketone), from PEKK (polyether ketone ketone) or from POM (polyoxymethylene), in addition preferably from ceramics, and the injection compound consists of a single material, preferably polyurethane, PEEK (polyether ether ketone), of PEKK (polyether ketone ketone) or of POM (polyoxymethylene), in addition preferably of ceramics or particularly preferably of the same materials as the supporting element (10).

10. The connection piece according to Claim 1, **characterised in that** the plug module (2) has a plug pin (2a) for contacting the electric implant, and has a contact pin (2b) for producing contact with a feed line of the electrode body, wherein the contact pin preferably has an abutment (2c), which cooperates with a hollow screw (16) screwed in the support element (10), such that the plug module (2) is fixed in the supporting element in the longitudinal direction (100).

11. An electrode line for cardiac or neural application, with a longitudinal axis, a proximal end and a distal end facing the therapy site, said electrode line comprising:
- a connection piece according to one of Claims 1 to 10 at the proximal end,
- a flexible and resilient electrode body with an electrically insulating outer sleeve, which extends from the proximal end to the distal end of the electrode body and in which n electric feed lines are embedded in an electrically insulated manner and produce an electric connection between the n electric connection lines (5a, 5b, 5c) with n electrode poles at the distal end,
- at least one coiled feed line, which is arranged over the longitudinal axis within the outer sleeve and which produces an electric connection between the electric plug module (2) and an electrode pole at the distal end, and
- a kink guard (23), which is fastened to the connection piece.

12. The electrode line according to Claim 11, **characterised in that** the electric connection and the simultaneous mechanical fastening between the electric connection lines (5a, 5b, 5c) and the embedded electric feed lines is produced by spring elements formed from electrically conductive material, which are fastened both to the electric connection lines (5a, 5b, 5c) and to the electric feed lines, wherein the spring elements are preferably crimpable and weldable wire coils, particularly preferably double coils.

13. The electrode line according to one of Claims 11 or 12, **characterised in that** the embedded feed lines and the coiled feed line are cables, particularly preferably DFT cables.

14. The electrode line according to one of Claims 12 to 13, **characterised in that** the feed lines, the spring elements and also the electrode poles as well as the conducting materials in the connection piece are fabricated from platinum-iridium alloys, tantalum, tantalum with a coating formed from a platinum-iridium alloy, elgiloy, or further implantable and conductive materials.

15. A method for producing a connection piece according to one of Claims 1 to 10, said method comprising the following steps:
- welding the electric connection lines (5a, 5b, 5c) to the electrically conducting contacts (3a, 3b, 3c),
- sliding the electrically conducting contacts alternately with the insulation portions (4a, 4b, 4c) onto the supporting element (10) as far as the stop (15) and aligning each electric connection line into one of the recesses (13a, 13b, 13c),
- filling the interior, delimited by the electrically conducting contacts (3a, 3b, 3c) and the insulation portions (4a, 4b, 4c, 4d) on the one hand and the supporting element (10) on the other hand, with an injection compound, and
- curing the injection compound.

16. A method for producing an electrode line according to one of Claims 11 to 14, said method comprising the following steps:
- connecting the contact pin (2b) to the coiled feed line,
- sliding the connection piece preassembled in accordance with Claim 15 onto the contact pin (2b),
- fastening the contact pin (2b) to the connection piece and welding the plug pin (2a) onto the contact pin (2b),
- crimping and/or welding the spring elements onto the embedded feed lines of the electrode body,
- sliding the flexible electrode body onto the coiled feed line,
- sliding and welding and/or crimping the spring element onto the electric connection lines (5a, 5b, 5c) of the connection piece, and
- sliding the kink guard (23) and latching in the kink guard on the stop (15).

17. The method according to Claim 16, **characterised in that** the contact pin (2b) is connected to the coiled feed line of the electrode body by a crimping and/or a welding process, and the contact pin is fastened to the connection piece by screwing in the hollow screw (16).

## Revendications

1. Pièce de raccordement (1) conçue comme une unité autonome destinée à être reliée à une ligne d'électrode et raccordée à un implant électrique, la pièce de raccordement présentant une forme étirée en longueur dans une direction longitudinale (100), ainsi qu'une extrémité proximale et une extrémité distale (10a, 10b), et comprenant:
- un module de prise (2) électriquement conducteur, faisant saillie dans la direction longitudinale (100) de façon proximale à partir de l'extrémité proximale de la pièce de raccordement,
- plusieurs contacts annulaires (3a, 3b, 3c) électriquement conducteurs et de même diamètre, avec des sections d'isolation (4a, 4b, 4c, 4d) de même diamètre, agencées entre ceux-ci dans la direction longitudinale, les sections d'isolation fixant les contacts électriquement conducteurs de manière à ce que leur position soit non modifiable dans la direction longitudinale (100),
- plusieurs lignes de raccordement électriques (5a, 5b, 5c) assurant une liaison de chacune des lignes d'alimentation correspondantes du corps d'électrode à l'extrémité distale de la pièce de raccordement, et
- un élément de support (10) avec une section transversale quasiment circulaire et avec un axe longitudinal le long de la direction longitudinale, lequel est conçu pour porter les sections d'isolation et pour les fixer dans la direction radiale (200), et lequel comporte un perçage traversant (11) situé sur son axe longitudinal et recevant le module de prise (2) et/ou une ligne d'alimentation d'un corps d'électrode,
dans laquelle
l'élément de support comporte n rainures périphériques (12a, 12b, 12c) espacées dans la direction longitudinale, et n évidements (13a, 13b, 13c) s'étendant parallèlement à l'axe longitudinal, de l'extrémité proximale à l'extrémité distale, pour guider des lignes de raccordement (5a, 5b, 5c) des contacts électriquement conducteurs (3a, 3b, 3c) vers l'extrémité distale, où n correspond au nombre de contacts électriquement conducteurs.

2. Pièce de raccordement selon la revendication 1, **caractérisée en ce que** les contacts électriquement conducteurs (3a, 3b, 3c) et les sections d'isolation (4a, 4b, 4c, 4d) d'une part et l'élément de support (10) d'autre part incluent un espace intérieur, dans lequel est introduite la masse d'injection par moulage par injection, après l'assemblage des contacts électriquement conducteurs, des sections d'isolation et de l'élément de support (10), reliant ainsi fixement entre eux les contacts électriquement conducteurs, les sections d'isolation et l'élément de support.

3. Pièce de raccordement selon la revendication 2, **caractérisée en ce que** l'espace intérieur est formé par des rainures périphériques (12a, 12b, 12c) et par les évidements (13a, 13b, 13c) de l'élément de support (10) s'étendant parallèlement à l'axe longitudinal, de l'extrémité proximale à l'extrémité distale.

4. Pièce de raccordement selon l'une des revendications 1 à 3, **caractérisée en ce que** les rainures périphériques (12a, 12b, 12c) sont espacées de telle façon les unes des autres, que les nervures périphériques (14a, 14b, 14c, 14d) situées avant et après les rainures dans la direction longitudinale portent les sections d'isolation (4a, 4b, 4c, 4d) et présentent un diamètre extérieur permettant un ajustement précis pour chacune des sections d'isolation.

5. Pièce de raccordement selon la revendication 4, **caractérisée en ce que** les nervures (14a, 14b, 14c, 14d) présentent une largeur de 1 à 6 mm, de préférence de 1,5 à 5,25 mm.

6. Pièce de raccordement selon la revendication 1 ou 3, **caractérisée en ce que** les évidements (13a, 13b, 13c) s'étendant le long de l'axe longitudinal sont ouverts radialement vers l'extérieur, de manière à ce que les lignes de raccordement électriques puissent être insérées facilement.

7. Pièce de raccordement selon la revendication 6, **caractérisée en ce que** les délimitations des évidements (13a, 13b, 13c) sont délimitées par des surfaces sécantes (17a, 17b, 17c) en direction de l'axe longitudinal, de sorte que l'élément de support (10) présente une section transversale polyédrique, de préférence avec un coin en N isocèle avec un cercle enveloppant comme section transversale.

8. Pièce de raccordement selon l'une des revendications 1 à 7, **caractérisée en ce que** l'élément de support (10) comporte une butée périphérique (15) à l'extrémité distale ou à proximité de celle-ci, pour permettre le montage exact des contacts électriques et des sections d'isolation.

9. Pièce de raccordement selon l'une des revendications 1 à 8, **caractérisée en ce que** l'élément de support (10) est fabriqué à partir d'un polymère, de préférence du PEEK (poly éther éther cétone), du PEKK (poly éther cétone cétone) ou du POM (polyoxyméthylène), de préférence également des céramiques, et la masse d'injection est constituée d'un seul matériau, de préférence du polyuréthane, du PEEK (poly éther éther cétone), du PEKK (poly éther cétone cétone) ou du POM (polyoxyméthylène), de préférence également des céramiques ou de manière particulièrement préférée à partir des mêmes matériaux que l'élément de support (10).

10. Pièce de raccordement selon la revendication 1, **caractérisée en ce que** le module de prise (2) comporte une broche de connecteur (2a) pour la mise en contact avec l'implant électrique, et une broche de contact (2b) pour l'établissement d'un contact avec une ligne d'alimentation du corps d'électrode, dans laquelle la broche de contact comporte de préférence une butée (2c) coopérant de telle façon avec une vis creuse (16) vissée dans l'élément de support (10), que le module de prise (2) est fixé dans l'élément de support dans la direction longitudinale (100).

11. Ligne d'électrode destinée à une utilisation cardiaque ou neuronale, avec un axe longitudinal, une extrémité proximale et une extrémité distale tournée vers le site de traitement, comprenant :
- une pièce de raccordement selon l'une des revendications 1 à 10 à l'extrémité proximale,
- un corps d'électrode souple et flexible, avec une enveloppe extérieur électriquement isolante, laquelle s'étend de l'extrémité proximale à l'extrémité distale du corps d'électrode, et dans laquelle n lignes d'alimentation électriques sont enrobées de façon électriquement isolée et établissent une liaison électrique entre les n lignes de raccordement électriques (5a, 5b, 5c) avec n pôles d'électrode à l'extrémité distale,
- au moins une ligne d'alimentation hélicoïdale située sur l'axe longitudinal à l'intérieur de l'enveloppe extérieure, laquelle établit une liaison électrique entre le module de prise (2) et un pôle d'électrode à l'extrémité distale, et
- une protection anti-courbure (23) fixée à la pièce de raccordement.

12. Ligne d'électrode selon la revendication 11, **caractérisée en ce que** la liaison électrique et la fixation mécanique simultanée entre les lignes de raccordement électriques (5a, 5b, 5c) et les lignes d'alimentation électriques enrobées sont réalisées par des éléments à ressort constitués d'un métal électriquement conducteur, lesquels sont fixés aussi bien aux lignes de raccordement électriques (5a, 5b, 5c) qu'aux lignes d'alimentation électriques, les éléments à ressort étant de préférence des fils hélicoïdaux sertissables ou soudables, et de manière particulièrement préférée des doubles fils hélicoïdaux.

13. Ligne d'électrodes selon l'une des revendications 11 ou 12, **caractérisée en ce que** les lignes d'alimentation enrobées et les câbles d'alimentation hélicoïdaux sont des câbles, de préférence des câbles TFD.

14. Ligne d'électrodes selon l'une des revendications 12 à 13, **caractérisée en ce que** les lignes d'alimentation, les éléments de ressort ainsi que les pôles d'électrode et les matériaux conducteurs dans la pièce de raccordement sont réalisés à partir d'alliages de platine-iridium, de tantale, de tantale avec un revêtement à base d'alliage de platine-iridium, d'Elgiloy, ou d'autres matériaux conducteurs implantables.

15. Procédé pour la fabrication d'une pièce de raccordement selon l'une des revendications 1 à 10, comprenant les étapes suivantes :
- soudage des lignes de raccordement électriques (5a, 5b, 5c) sur les contacts électriquement conducteurs (3a, 3b, 3c),
- enfilement des contacts électriquement conducteurs en alternance avec les sections d'isolation (4a, 4b, 4c, 4d) sur l'élément de support (10) jusqu'à la butée (15), et orientation de chacune des lignes de raccordement électriques dans l'un des évidements (13a, 13b, 13c),
- remplissage de l'espace intérieur délimité d'une part par les contacts électriquement conducteurs (3a, 3b, 3c) et les sections d'isolation (4a, 4b, 4c, 4d) et d'autre part par l'élément de support (10), avec une masse injectée, et
- durcissement de la matière injectée.

16. Procédé pour la fabrication d'une ligne d'électrodes selon l'une des revendications 11 à 14, comprenant les étapes suivantes :
- liaison de la broche de contact (2b) avec la ligne d'alimentation hélicoïdale,
- enfilement de la pièce de raccordement prémontée selon la revendication 15 sur la broche de contact (2b),
- fixation de la broche de contact (2b) sur la pièce de raccordement et soudage de la broche de connecteur (2a) sur la broche de contact (2b),
- sertissage et/ou soudage des éléments à ressort sur les lignes d'alimentation enrobées du corps d'électrode,
- enfilement du corps d'électrode souple sur la ligne d'alimentation hélicoïdale,
- enfilement et soudage et/ou sertissage de l'élément à ressort sur les lignes de raccordement électriques (5a, 5b, 5c) de la pièce de raccordement, et
- enfilement de la protection anti-courbure (23) et enclenchement de la protection anti-courbure sur la butée (15).

17. Procédé selon la revendication 16, **caractérisé en ce que** la liaison entre la broche de contact (2b) et l'alimentation hélicoïdale du corps d'électrode est réalisée par une opération de sertissage et/ou de soudage, et la fixation de la broche de contact sur la pièce de raccordement est réalisée par vissage de la vis creuse (16).
